# EUROPEAN PATENT SPECIFICATION

(11) **EP 3 934 500 B1**
(45) Date of publication and mention of the grant of the patent: **05.04.2023**
(21) Application number: 20716223.1
(22) Date of filing: 05.03.2020
(51) Int. Cl.: A47L 7/00, A47L 11/03, A47L 11/20, A47L 11/30

(54) **MULTIFUNCTION MACHINE FOR CLEANING AND SANITIZING SURFACES AND ENVIRONMENTS**
MULTIFUNKTIONSMASCHINE ZUM REINIGEN UND DESINFIZIEREN VON OBERFLÄCHEN UND UMGEBUNGEN
MACHINE MULTIFONCTION SERVANT À NETTOYER ET À DÉSINFECTER DES SURFACES ET DES ENVIRONNEMENTS

(30) Priority: 05.03.2019 IT 201900003187
(43) Date of publication of application: 12.01.2022
(73) Proprietor: T.P.A. IMPEX S.p.A., 36060 Romano d'Ezzelino (VI) (IT)
(72) Inventor: AMORETTI, Luigi, 36065 Mussolente (IT)
(74) Representative: GCA S.R.L.
(86) International application number: PCT/IT2020/050051
(87) International publication number: WO 2020/178872

(56) References cited:
- EP-A1- 1 629 759
- EP-A2- 1 330 976
- EP-B1- 1 330 976
- EP-B1- 1 629 759
- DE-A1- 19 933 180
- DE-C2- 19 933 180

## Description

### Field of the invention

The invention concerns a multifunction machine for cleaning and sanitizing surfaces and environments, which can generally be used to perform cleaning and sanitizing operations with a single machine that contains all the devices necessary to complete the operations.

### Background of the invention

In the sector of machines for cleaning and sanitizing environments and surfaces, both domestic and also industrial, multifunction machines are known which comprise a series of devices combined together in a single body-machine and which allow users to perform a series of cleaning and sanitizing works, either using the various devices individually, or using them in combination with each other.

A multifunctional machine for cleaning surfaces is described in patent application WO2018146710, which specifically concerns a multifunction vacuum cleaner which comprises a body-machine, inside which there is defined a path for the passage of flows of sucked air and debris carried in suspension in the flows of sucked air.

The air and debris are bubbled inside a tank loaded with a liquid, typically water, in order to release the sucked particles of debris into it.

After passing in this tank, which in practice is a water filter, the flows of air are sent by the internal path into the housing seating of the motor of the vacuum cleaner so as to be used to cool it, before being definitively expelled outside the body-machine, into the surrounding environment.

In this multifunctional vacuum cleaner there is also provided a steam production unit which can be activated on command, for example when the cleaning operations are particularly difficult in the presence of stubborn dirt.

The steam is used, together with the suction action, to dissolve the dirt which, after being detached from the surface to be cleaned, is then sucked in, in a conventional manner.

In this multifunctional vacuum cleaner there is also provided the installation, as an accessory, of a device for the diffusion of perfumes into the air to make the environments in which the vacuum cleaner is used more pleasant.

From patent EP1330976 a vacuum cleaner is known that has an ion generator.

According to this patent, the vacuum cleaner comprises a suction path of air and debris that starts with a flexible suction pipe that is connected to a motor-unit that drives a fan, which is able to create the depression for the suction action.

Upstream of the motor-unit there is provided a debris collection chamber in which a paper bag is housed, in which the latter are collected.

The flow of air sucked in is then filtered by a primary and a secondary filter, before being released back into the atmosphere.

The ion generator can be positioned both directly on the suction nozzle with which the suction pipe is equipped, and also immediately downstream of the motor-unit, before the flow of air sucked in exits from the body-machine.

From EP1629759 a "Domestic vacuum cleaner" is known, which comprises a body adapted to comprise suction means in fluid communication with an air intake opening, filtering means to filter the particles contained in the suctioned air. The filtering means are interposed between the intake and the suction means.

The vacuum cleaner further comprises an air discharge path to convey a flow of air suctioned by the suction means to an outlet opening a tank for a fluid associated with the body in order to be interposed in fluid communication between the suction means and the outlet. The vacuum cleaner is characterized by comprising a first air discharge path in order to convey a first flow of air discharged by the suction means to an outlet opening and a second air discharge path in order to convey a second flow of air discharged by the suction means to means for generating an atomized flow, so that the first flow of air suctioned by the suction means is intercepted by the atomized flow in the first air discharge path so as to extract the particles contained in the suctioned air.

In DE19933180 it is disclosed a process for de-dusting, deodorizing and sterilizing air, which comprises enriching the air with active oxygen and ozone using ionizers electrically connected before a filter medium in the flow direction. The ionization component groups are connected with the filter medium so that when the filter medium is exchanged, the ionization component groups must be exchanged. The electrical connection to a high voltage generator occurs via electrical contacts on the filter medium.

This state of the art has some disadvantages.

One disadvantage relating to patent application WO2018146710 is that a device is not provided which, in the multifunction vacuum cleaner, can generate and emit both negative and also positive ions.

This limits the performance obtainable with this multifunction vacuum cleaner, in particular in terms of purification and deodorization of the air sucked in.

A second disadvantage specifically related to patent EP1330976 is that with this vacuum cleaner that generates ions, it is not possible to suck in liquids.

Furthermore, in both cases, there is only one passage path of the sucked in air and, therefore, in the case of ion generation, it is not possible to separate the generation and emission path from the specific path for sucking in debris.

For this reason, these known vacuum cleaners cannot be equipped with water filtration or be suitable to suck in, as well as dry debris, also liquids which are carrying debris.

In addition, it happens that the ions that are generated and emitted in the same suction circuit of known vacuum cleaners, are partly or totally covered with debris sucked in with the air, significantly reducing their effects of sanitizing the environment.

### Purposes of the invention

One purpose of the invention is to improve the state of the art.

Another purpose of the invention is to provide a machine for cleaning and sanitizing surfaces and environments that is able to produce ions and, at the same time, also suck in liquids without creating dangers for the safety of users.

According to one aspect of the invention, a machine is provided for cleaning and sanitizing surfaces and environments, in accordance with the characteristics of claim 1.

Other characteristics of the invention are included in the dependent claims. The invention allows to obtain the following advantages:
- obtain a machine for cleaning and sanitizing surfaces and environments that is equipped with a number of functions that can be used both separately and also in combination with each other;
- obtain a machine for cleaning and sanitizing surfaces and environments that allows to suck in debris that are solid or even liquid or immersed in a liquid, generating positive or negative ions according to requirements;
- eliminate the need to use container bags to accumulate the debris and the dirt sucked into a multifunction machine able to generate ions.

### Brief description of the drawings

Other characteristics and advantages of the invention will become more apparent from the detailed description of preferred, but not exclusive, embodiments of a machine for cleaning and sanitizing surfaces and environments, given as a non-restrictive example in the attached drawings wherein:
Fig. 1 is a very schematic and cut-away view of the machine for cleaning and sanitizing surfaces and environments according to the invention;
Fig. 2 is a detailed view of a part of the machine of fig. 1;
Fig. 3 is a detailed view on a slightly enlarged scale of a portion of an ion release element with which the machine is equipped to clean and sanitize surfaces and environments according to the invention;
Fig. 4 is another very schematic and cut-away view of a second version of the machine for cleaning and sanitizing surfaces and environments according to the invention.

### Detailed description of a preferred example embodiment

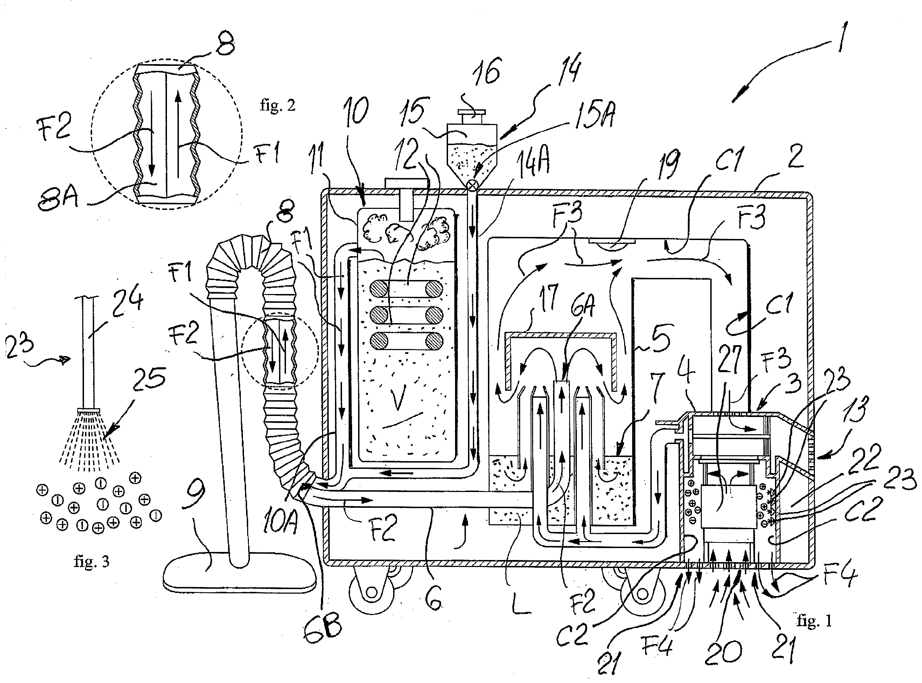

With reference to fig. 1, 1 indicates a machine for cleaning and sanitizing surfaces and environments according to the invention, and indicated hereafter as machine 1 in brief.

The machine 1 is preferably, but not exclusively, made in the form of a vacuum cleaner household appliance and has a box-like body-machine 2 inside which a number of devices are assembled to perform a corresponding number of operating functions.

In detail, the machine 1 comprises a suction group, typically a motor 3 which is housed inside a containment casing 4 and which is connected to a tank 5 of liquid L by means of a primary circuit C1.

The tank 5 is only partly filled with the liquid L up to a level line 7 and which is an embodiment both of wet filtration means, and also of means for collecting sucked in debris.

Into the upper part of the tank 5 flows the end 6A of a conduit 6 which defines one end of the conduit 6 while the opposite end 6B thereof is provided with a mouth for engaging a conventional flexible tube 8 supplied with the machine 1 and which connects the opposite end 6B to a terminal mouth 9.

Inside the body-machine 2 there is also housed, indicated as a whole with 10, a unit to produce steam and to expel it on command toward surfaces and environments to be cleaned and sanitized.

The unit 10 comprises a second tank 11 in which resistances 12 able to be heated are arranged, the whole typically forming a boiler, and a volume V of water, to generate steam when required during the use of the machine 1.

The steam produced is conveyed in the direction of the arrow F1 toward the mouth 9 by means of an independent conduit 10A which extends inside the flexible tube 8, parallel thereto, but separated from the conduit 8A through which both the sucked in air (arrow F2) and also the sucked in debris which are suspended therein, pass from the outside toward the inside of the body-machine 2.

The tank 5 and the casing 4 are connected to each other by means of the primary circuit C1 in which sucked in air circulates (arrows F3) after being purified from the debris that, having exited from the end 6A, were released into the liquid L contained in the tank 10.

The primary circuit C1 has a first inlet which consists of the same end 6B of the conduit 6, and an outlet 13 for expelling the air toward the outside, which is made in the body-machine 2.

Another tube 14A also converges in the tube 8 which connects a device 14 for accumulating and emanating perfumes, aromas or disinfectants, preferably prepared in a nebulized or gaseous form, which are loaded into a further third tank 15 which the device 14 is equipped with, and which is equipped with a loading aperture 16 and which is associated with the body-machine 2.

The other tube 14A is controlled by a valve 15A with two alternative positions of use, precisely open or closed.

Inside the tank 5 there are also mounted a deflector device 17 and an emitting source 19 of UV or UV-C rays.

In the body-machine 2 there is also defined a secondary circulation circuit C2 for air, which has a second inlet 20 and a second outlet 21 and inside which flows of air F4 flow, sucked in by a fan 27 which is driven in rotation by the same motor 3 but which has significantly smaller sizes than the latter, so as to generate a depression with an absolute value lower than that generated by the motor 3 in the primary circuit C1.

An ionizing device 22 is installed in the secondary circuit C2, structurally known to the person of skill in the art, which comprises a plurality of elements 23 for the delivery of ions, both positive and negative, and which can be activated on command by a user.

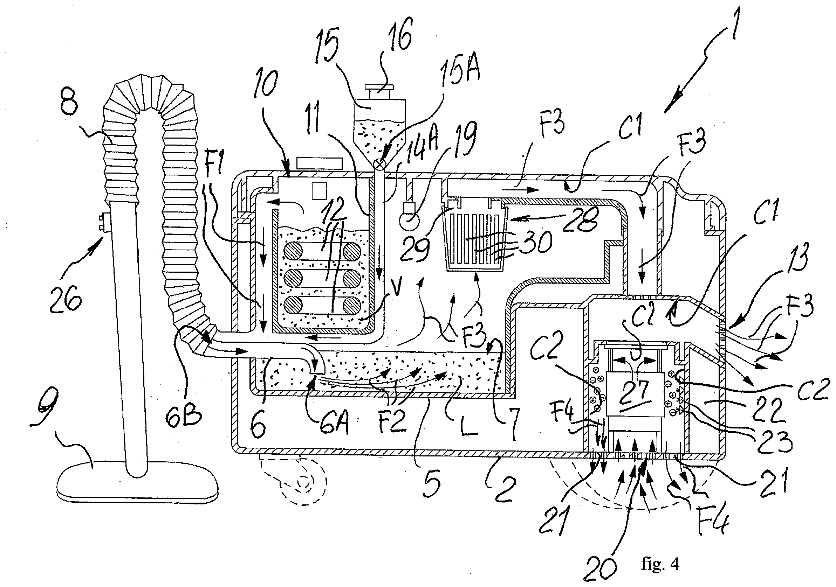

With reference to fig. 4, in which for convenience elements common to those of fig. 1 have been indicated with the same numerical references, it can be noted that, inside the body-machine 2, there are again the motor 3 housed in its own containment casing 4, the tank 5 in which the liquid L is accumulated up to the level line 7, the secondary circuit C2 in which, during the functioning of the machine 1, a depression is obtained from the action of the fan 27 driven by the motor 3, the ionizing device 22 and the corresponding ion delivery elements 23.

In this second version of the machine 1 there is also still the conduit 6, at the end 6B of which there is connected the flexible tube 8 with its terminal mouth 9.

The steam production unit 10 is also incorporated in the body-machine 2 while, compared to the previous version, inside the tank 5 there is provided the assembly of a molecular separator device 28, intended to further filter the flows of air F3 that exit from the liquid L, eliminating from them even the particles of very small sizes that had possibly remained in suspension after the bubbling in the liquid L.

The molecular separator device 28 comprises a rotating fan 29, which is arranged in a support casing, which can be equipped with a spongy perimeter filter, and which is driven by the depression generated by the motor 3 inside the tank 5 through the primary circuit C1.

The rotating fan 29 has a plurality of passage apertures 30, through which the flows of air F3 pass after having released the debris sucked into the liquid L by gravity or due to impacts against the rotating fan 29 itself.

The molecular separator 28, when in the tank 5, therefore provides to dynamically separate the residual debris from the flows F3 of air sucked in, acting practically as a filter, so that the flows of filtered air F3, after having passed through the apertures 30, can first reach the inside of the casing 4 and lap the motor 3, cooling it, and subsequently be expelled completely purified toward the external environment, through the first outlet 13.

With reference to fig. 3, it can be noted in detail that each element 23 comprises a thin electrical conduit 24 for connection with the ionizing device 22, which ends with a dilated end 25 and preferably, but not exclusively, with a truncated-cone or brush-like shape which is made with a bunch of thin bristles, preferably made of carbon fiber, which release ions into the environment when they are lapped by the flows of air F4.

It should be noted that the primary circuit C1 and the secondary circuit C2 are made in such a way as to always be separated from each other since, for functioning safety and for the safety of users, the ionizing device 22, which is positioned along the secondary circuit C2, must not have any point of contact with the liquid L.

The functioning of the multifunction machine for cleaning and sanitizing surfaces and environments is described below.

In the first embodiment of the multifunction machine 1 shown in fig. 1, when only the suction action is required, a user activates the motor 3 which provides to put the tank 5 into depression and to generate the suction action which is transmitted to the outside through the flexible tube 8, which transports inside the tank 5 all the sucked in debris which are in suspension in the flows F2 of sucked in air.

The flows F2 of sucked in air exit from the end 6A of the conduit 6 and bubble inside the liquid L below the level line 7, releasing inside it most of the sucked in debris, typically those that have a greater weight, which gather on the bottom of the tank 5.

The flows F3 of air purified from most of the coarser debris are directed by Venturi effect toward the deflector device 17 which creates a turbulence in the part of the tank 5 not occupied by the liquid L, and due to which the particles of debris possibly still remaining in suspension fall back into the latter.

The deflector device 17 deflects the flows of air F3 toward the motor 3, through the primary circuit C1, so that these can cool it, lapping it, to then be expelled toward the outside through the first outlet 13.

During the functioning of the machine 1, the user, faced with particularly tenacious dirt on a surface to be cleaned and sanitized, can activate the unit 10 for producing steam which, through the independent conduit 10A, passes inside the flexible tube 8 until it is expelled from the terminal mouth 9 toward the surface.

Also during the functioning of the machine 1, the user can also activate the ionizing device 22 which generates positive or negative ions which are introduced into the surrounding environment, passing into the secondary circuit C2 and exiting from the second outlet 21, without lapping in any way the liquid L contained in the tank 5, therefore in conditions of maximum safety.

Also during the functioning of the machine 1, when required, the user can open the valve 15A and expel from the terminal mouth 9, together with the steam generated by the steam generation unit 10, also deodorant or disinfectant substances.

The functioning of the second version of the machine 1 shown in fig. 4 is substantially the same as that described for the first version, with the only difference that the flows F3 of air exiting the liquid L are sucked toward the apertures 30 of the molecular separator 28 which, by rotating, abates from the flows F3 the residual debris which may possibly have remained in suspension therein, due to their very small sizes and weights.

It should be noted that in both versions of the machine 1, during the passage between the level line 7 of the liquid L and the deflector device 17 or the molecular separator 28, the flows F3 of air are subjected to the antibacterial sanitizing action provided by the source 19 of UV or UV-C rays, preferably arranged on the top of the tank 5.

Ultimately, the multifunction machine according to the invention is able to produce and emit ions through only the secondary circuit C2, while the primary circuit C1 is used for the suction of debris and dirt, both dry and also contained in liquids sucked in.

In practice it has been verified that the invention achieves the intended purposes, that is to combine in a single multifunction machine a plurality of devices, each of which provides a specific performance.

A person of skill in the art understands that the devices incorporated in the multifunction machine 1 can be driven both independently of one another, and also simultaneously, even only partly.

The invention as conceived is susceptible to modifications and variants, all of which are within the scope of the inventive concept.

Furthermore, all the details can be replaced with other technically equivalent elements.

In their practical embodiment, any other materials, as well as shapes and sizes, can be used according to requirements, without departing from the main field of protection, which is defined by the following claims.

## Claims

1. Multifunction machine (1) for cleaning and sanitizing surfaces and environments that has a body-machine (2) that includes:
- a waste suction group (3);
- wet filtration and collection means (5) of sucked waste;
- a steam generating and steam expulsion control unit (10) towards said surfaces and environments;
- a primary circuit (C1) for circulating sucked air and waste having a first inlet (6B) and a first outlet (13) obtained in said body-machine (2);
**characterized in that** said body-machine further includes:
- a secondary circulation circuit (C2) for air which has a second inlet (20) and a second outlet (21) obtained in said body-machine (2); and
- an ionizing device (22) placed on said secondary circuit (C2) between said second inlet (20) and second outlet (21).

2. The machine according to claim 1, wherein said ionizing device (22) comprises pointed delivery elements (23) located at said second outlet (21) for the delivery of ions into the environment.

3. The machine according to any one of the preceding claims, wherein said delivery elements (23) each comprises an electrical supply conduit (24) which has one end connected to said ionizing device (22) and an opposite free end that comprises a plurality of bristles (25) each of which is designed to release ions.

4. The machine according to claim 1, wherein said primary circuit (C1) and secondary circuit (C2) are separated from each other.

5. The machine according to claim 1, wherein it further comprises a molecular separator device (28) arranged inside said wet filtration and collection means (5) of sucked waste.

6. The machine according to claim 1, wherein it further comprises an emitting source (19) of UV, or UV-C rays.

## Patentansprüche

1. Multifunktionsmaschine (1) zum Reinigen und Desinfizieren von Oberflächen und Umgebungen, die eine Körpermaschine (2) hat, die umfasst:
- eine Abfallansauggruppe (3);
- Nassfiltrations- und -sammelmittel (5) für den angesaugten Abfall;
- eine Dampferzeugungs- und Dampfausstoß-Steuerungseinheit (10) hin zu den genannten Oberflächen und Umgebungen;
- einen Primärkreislauf (C1) zum Zirkulieren von angesaugter Luft und Abfall, der einen ersten Einlass (6B) und einen ersten Auslass (13) hat, die in der genannten Körpermaschine (2) ausgebildet sind;
**dadurch gekennzeichnet, dass** die genannte Körpermaschine ferner umfasst:
- einen Sekundärzirkulationskreislauf (C2) für die Luft, der einen zweiten Einlass (20) und einen zweiten Auslass (21) hat, die in der genannten Körpermaschine (2) ausgebildet sind; und
- eine Ionisierungsvorrichtung (22), die an dem genannten Sekundärkreislauf (C2) zwischen dem genannten zweiten Einlass (20) und dem zweiten Auslass (21) gelegen ist.

2. Maschine nach Anspruch 1, worin die Ionisierungsvorrichtung (22) spitze Zufuhrelemente (23) umfasst, die am genannten zweiten Auslass (21) für die Zufuhr von Ionen in die Umgebung gelegen sind.

3. Maschine nach einem der vorhergehenden Ansprüche, worin die genannten Zufuhrelemente (23) je eine elektrische Versorgungsleitung (24) umfassen, die ein Ende, das mit der genannten Ionisierungsvorrichtung (22) verbunden ist, und ein entgegengesetztes freies Ende, das eine Vielzahl von Borsten (25) umfasst, von denen jede dafür ausgelegt ist, Ionen abzugeben, aufweist.

4. Maschine nach Anspruch 1, worin der genannte Primärkreislauf (C1) und der Sekundärkreislauf (C2) voneinander getrennt sind.

5. Maschine nach Anspruch 1, worin sie ferner eine Molekülseparatorvorrichtung (28) umfasst, die innerhalb der genannten Nassfiltrations- und -sammelmittel (5) für den angesaugten Abfall angeordnet ist.

6. Maschine nach Anspruch 1, worin sie ferner eine Emissionsquelle (19) von UV- oder UV-C-Strahlen umfasst.

## Revendications

1. Machine multifonctionnelle (1) pour le nettoyage et l'assainissement de surfaces et d'environnements, qui comporte une machine-corps (2) qui comprend :
- un groupe d'aspiration de déchets (3) ;
- des moyens de filtration et de collecte à voie humide (5) des déchets aspirés ;
- une unité de commande de génération de vapeur et d'expulsion de vapeur (10) vers lesdites surfaces et lesdits environnements ;
- un circuit primaire (C1) pour faire circuler l'air aspiré et les déchets ayant une première entrée (6B) et une première sortie (13) obtenues dans ladite machine-corps (2) ;
**caractérisée en ce que** ladite machine comprend en outre :
- un circuit de circulation secondaire (C2) pour l'air qui comporte une seconde entrée (20) et une seconde sortie (21) obtenues dans ladite machine-corps (2) ; et
- un dispositif d'ionisation (22) placé sur ledit circuit secondaire (C2) entre ladite seconde entrée (20) et ladite seconde sortie (21).

2. Machine selon la revendication 1, dans laquelle ledit dispositif d'ionisation (22) comprend des éléments de distribution pointus (23) situés au niveau de ladite seconde sortie (21) pour la distribution d'ions dans l'environnement.

3. Machine selon l'une quelconque des revendications précédentes, dans laquelle lesdits éléments de distribution (23) comprennent chacun un conduit d'alimentation électrique (24) qui a une extrémité reliée audit dispositif d'ionisation (22) et une extrémité libre opposée qui comprend une pluralité de soies (25), chacune desquelles est conçu pour libérer des ions.

4. Machine selon la revendication 1, dans laquelle ledit circuit primaire (C1) et ledit circuit secondaire (C2) sont séparés l'un de l'autre.

5. Machine selon la revendication 1, dans laquelle elle comprend en outre un dispositif de séparation moléculaire (28) agencé à l'intérieur desdits moyens de filtration et de collecte à voie humide (5) de déchets aspirés.

6. Machine selon la revendication 1, **caractérisée en ce qu'**elle comprend en outre une source émettrice (19) de rayons UV ou UV-C.
